# EUROPÄISCHE PATENTSCHRIFT

(11) **EP 1 755 730 B1**
(45) Veröffentlichungstag und Bekanntmachung des Hinweises auf die Patenterteilung: **24.03.2010**
(21) Anmeldenummer: 05759098.6
(22) Anmeldetag: 15.06.2005
(51) Int. Cl.: A61M 39/24, F16K 15/14

(54) **RÜCKSCHLAGVENTIL, INSBESONDERE FÜR MEDIZINISCHE ANWENDUNGEN**
NON-RETURN VALVE, PARTICULARLY FOR MEDICAL APPLICATIONS
CLAPET ANTIRETOUR, NOTAMMENT A USAGE MEDICAL

(30) Priorität: 17.06.2004 DE 202004009521 U
(43) Veröffentlichungstag der Anmeldung: 28.02.2007
(73) Patentinhaber: Filtertek B.V., County of Limerick (IE)
(72) Erfinder: MIJERS, Jan, Willem, Marinus, N-2101 EH Heemstede (NL); HOGAN, Brendan, Gort (IE)
(74) Vertreter: Brose, D. Karl
(86) Internationale Anmeldenummer: PCT/EP2005/006425
(87) Internationale Veröffentlichungsnummer: WO 2005/123176

(56) Entgegenhaltungen:
- EP-A- 0 612 537
- DE-U1-202004 009 521
- GB-A- 439 278
- US-A- 4 188 978

## Beschreibung

Die Erfindung betrifft ein Rückschlagventil, insbesondere für medizinische Anwendungen, mit einem ersten Schlauchanschlussgehäuse und einem zweiten Schlauchanschlussgehäuse und einer zwischen beiden Schlauchanschlussgehäusen angeordneten Membranscheibe aus flexiblem Werkstoff, welche bei Überdruck in einem Eingangsdurchlass im ersten Schlauchanschlussgehäuse von einem ringförmig, einen mit dem Eingangsdurchlass verbundenen Einlassraum umgebenden Ventilsitz abhebbar ist und die bei Überdruck in einem Ausgangsdurchlass sicher und in Minimalzeiten auf den Ventilsitz andrückbar ist, wobei die Membranscheibe an ihrem äusseren Umfangsbereich mit einem Ringwulst versehen ist, welcher in einander gegenüber liegenden Ringnuten der Schlauchanschlussgehäuse aufgenommen ist, wobei die Membranscheibe radial ausserhalb des Ventilsitzes mit Öffnungen versehen ist, die mit einem Auslassraum verbunden sind.

Ein derartiges Rückschlagventil ist nach einem früheren Vorschlag der Anmelderin aus dem europäischen Patent 0 612 537 bekannt.

In der Medizintechnik werden derartige Rückschlagventile für die Leitungen von Infusionssystemen, Spritzen, Diagnoseausrüstungen, intravenösen Schlauchleitungen und in Verbindung mit Spritzpumpen verwendet. Derartige Rückschlagventile müssen bei Schließzeiten von wenigen Bruchteilen von Sekunden sicher schließen, um jeden Rückfluss von möglicherweise kontaminierten Flüssigkeiten zu vermeiden. Da derartige Rückschlagventile in der Medizintechnik lediglich als Einwegartikel verwendet werden, muss gleichzeitig die Herstellung einerseits extrem wirtschaftlich und andererseits statistisch sehr genau sein. Darüber hinaus sind sehr strenge gesetztliche Vorschriften und Untersuchungen vorgesehen, um eine gleichbleibende einheitliche Funktionssicherheit sicherzustellen. Dies wird in Deutschland beispielsweise durch den TÜV genau geprüft, ehe nach Erreichen positiver Ergebnisse die Freigabe für die medizinische Anwendung erfolgt. Diese Anforderungen wurden bei dem oben erwähnten Rückschlagventil nach dem früheren Vorschlag der Anmelderin durchaus erfüllt, da bei dieser bekannten Konstruktion die den Ringwulst am Umfangsbereich der Membran aufnehmenden Ringnuten derart gestaltet sind, dass bei der Montage der beiden Schlauchanschlussgehäuse radial gerichtete Zugkräfte auf die Membranscheibe aufgebracht werden, so dass hierdurch eine Einstellung der Zugspannung in der Membran erfolgen kann, welche für ein ausgesprochen schnelles und sicheres Ansprechen des Rückschlagventils sorgt.

Es hat sich jedoch beim praktischen Einsatz dieses bekannten Ventils gezeigt, dass es wünschenswert ist, mit geringem Aufwand Ventile bereitstellen zu können, die höhere Öffnungsdrücke aufweisen. Beispielsweise bei der Verwendung im Zusammenhang mit Spritzpumpen muss sichergestellt werden, dass durch den Höhenunterschied zwischen Patient und Spritzpumpe diese nicht einfach leerlaufen kann, da das Ventil bereits aufgrund der geodätischen Höhe öffnen könnte. Da derartige Ventile jedoch üblicherweise aus Spritzgussteilen hergestellt werden, ist die Herstellung von Ventilen mit unterschiedlichen Öffnungsdrücken mit erheblichen Formkosten belastet.

Der Erfindung liegt daher die Aufgabe zugrunde, Ventile der eingangs definierten Art derart zu verbessern, dass mit einfachsten Mitteln unterschiedliche Öffnungsdrücke eingestellt werden können.

Bei einem Ventil der eingangs definierten Art wird diese Aufgabe erfindungsgemäß im Wesentlichen dadurch gelöst, dass auf der der Membranscheibe gegenüber liegenden Wandung des zweiten Schlauchanschlussgehäuses eine an der dem Auslassraum zugewandten Seite der Membranscheibe anliegende Formation vorgesehen ist, welche die Membranscheibe in Richtung des Einlassraums hält bzw. vorspannt, dass die Formation innerhalb der durch den Ventilsitz gegebenen Grenzen angeordnet ist, und dass dur Öffnungsdruck des Ventils durch den Abstand Zwischen der Formation und der Vertilsitz bestimmt ist.

Es ist offensichtlich, dass durch die durch die Formation gegebene Abstützung der Membranscheibe innerhalb des Ventilsitzes bzw. durch die Größe der durch die Formation in der Membranscheibe erzeugte gegensinnige Vorspannung eine einfache Möglichkeit geschaffen wird, Ventile unterschiedlichster Öffnungsdrücke herzustellen, indem entweder der Radius, auf welchem derartige Formationen innerhalb des Ventilsitzes liegen, oder die Höhe der Formationen und somit die Größe der gegensinnigen Vorspannung geändert wird. Dies lässt sich unter geringen Formkasten leicht dadurch erreichen, dass lediglich eine Hälfte der zur Herstellung des zweiten Schlauchanschlussgehäuses verwendeten Spritzgießform entsprechend geändert wird.

Bei einer bevorzugten Ausführungsform der Erfindung ist die Formation mediendurchlässig ausgebildet, d.h. dass sie die Strömung durch das geöffnete Ventil kaum behindert.

Eine besonders bevorzugte Ausführungsform nach der Erfindung kann dadurch geschaffen werden, dass die Formation durch eine Anzahl von Vorsprüngen gebildet ist, welche kronenförmig die Eingangsöffnung des Ausgangsdurchlasses vom Auslassraum umgeben.

Ferner ist es bevorzugt, die Vorsprünge einstückig mit dem zweiten Schlauchanschlussgehäuse auszubilden.

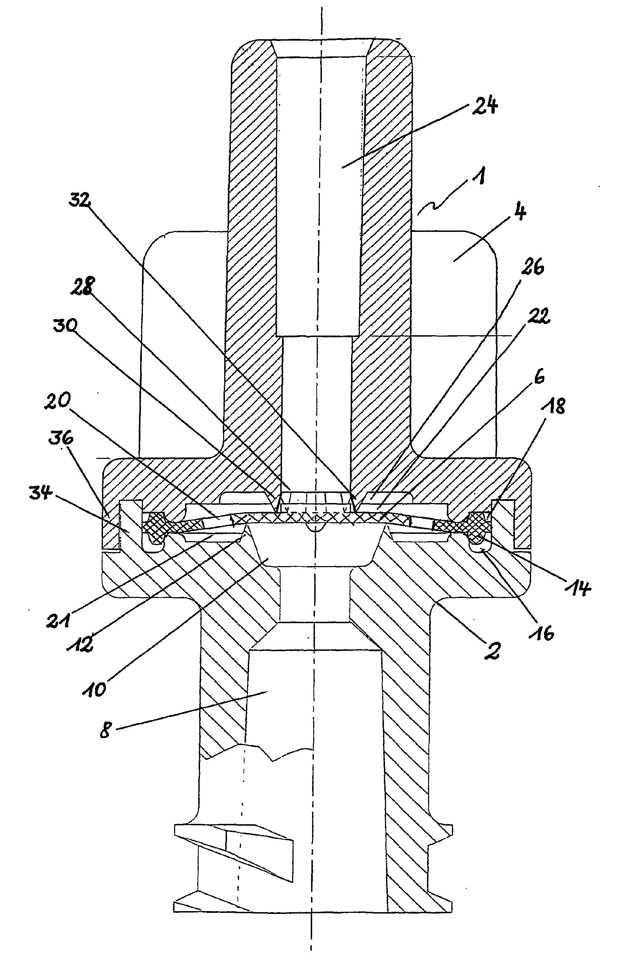

Im Folgenden wird die Erfindung an Hand einer in der Zeichnung beispielhaft veranschaulichten Ausführungsform näher erläutert. Die Zeichnung zeigt eine Schnittansicht des erfindungsgemäßen Rückschlagventils in einer möglichen Ausführungsform im fertig montierten Zustand.

Das in der Zeichnung gezeigte Rückschlagventil 1 ist insbesondere für medizinische Zwecke geeignet und deckt beispielsweise Öffnungsdrücke zwischen 0,14 und 0,42 bar ab, während Rückschlagventile der eingangs definierten Art nach dem früheren Vorschlag der Anmelderin für Druckunterschiede zwischen 0,1 bis 0,002 bar konzipiert sind.

Das Rückschlagventil 1 besteht aus einem ersten Schlauchanschlussgehäuse 2 und einem zweiten Schlauchanschlussgehäuse 4, welche beispielsweise durch Spritzgießen aus Kunststoff hergestellt sind und einer zwischen diesen eingespannten Membranscheibe 6. Die Membranscheibe 6 besteht aus einem flexiblen Werkstoff, beispielsweise Silikon.

Das erste Schlauchanschlussgehäuse 2 weist einen Eingangsdurchlass 8 auf, welcher in einem Einlassraum 10 mündet. Der Einlassraum 10 ist von einem ringförmigen Ventilsitz 12 umgeben, gegen den die Membran 6 vorgespannt ist.

Die Membran 6 ist in ihrem innerhalb des Ventilsitzes 12 liegenden Bereich durchgehend ausgebildet, so dass erhebliche Zugkräfte radial von innen nach aussen und umgekehrt übertragen werden können. An ihrem äusseren Umfangsbereich ist die Membran 6 mit einem Ringwulst 14 versehen, welcher dort beispielsweise an die Membran 6 angespritzt ist, falls diese ebenfalls durch Spritzgießen des Silikons hergestellt ist. In dem ersten Schlauchanschlussgehäuse 2 ist in dessen Stirnfläche eine Ringnut 16 ausgebildet, wobei eine Ringnut 18 des zweiten Schlauchanschlussgehäuses 4 dieser im zusammengebauten Zustand gegenüber liegt. Beim Zusammenbau des ersten Schlauchanschlussgehäuses mit dem zweiten Schlauchanschlussgehäuse wird der Ringwulst 14 in die einander gegenüber liegenden Ringnuten 16 und 18 der beiden Schlauchanschlussgehäuse 2 und 4 aufgenommen und die Membranscheibe 6 gleichzeitig gegen den Ventilsitz 12 vorgespannt.

Wie ferner aus der Zeichnung ersichtlich, ist die Membranscheibe 6 radial ausserhalb des Ventilsitzes 12 mit auf einem Radius angeordneten Öffnungen 20 versehen, welche einen radial ausserhalb des Ventilsitzes 12 liegenden Ringraum 21 im ersten Schlauchanschlussgehäuse 2 mit einem Auslassraum 22 des zweiten Schlauchanschlussgehäuses 4 verbinden, der seinerseits mit dem Ausgangsdurchlass 24 des zweiten Schlauchanschlussgehäuses 4 in Verbindung steht.

Die Wandung 26 des zweiten Schlauchanschlussgehäuses 4 liegt der Membranscheibe 6 gegenüber und begrenzt den Ausgangsraum 22 nach oben. An der Wandung 26 sind allgemein mit 28 bezeichnete vorstehende Formationen, welche mediendurchlässig sind, vorgesehen. Die Formationen 28 stützen die Membranscheibe 6 ab bzw. spannen die Membranscheibe 6 in Richtung des Einlassraumes 10 vor. Wie gezeigt, liegt die Formation 28 innerhalb der durch den Ventilsitz 12 gegebenen Grenze.

Bei dem veranschaulichten Ausführungsbeispiel ist die Formation 28 durch eine Anzahl von Vorsprüngen 30 gebildet. Die Vorsprünge 30 umgeben in Form einer Krone die vom Auslassraum 32 ausgehende Eingangsöffnung 32 des Ausgangsdurchlasses. Wie gezeigt, sind die Vorsprünge 30 durch Spritzgießen einstückig mit dem zweiten Schlauchanschlussgehäuse 4 ausgebildet. Es ist offensichtlich, dass durch die Höhe der Vorsprünge 30 die auf die Membranscheibe 6 ausgeübte gegensinnige Vorspannung geändert werden kann, was selbstverständlich eine Änderung des Öffnungsdrucks des Rückschlagventils 1 mit sich bringt. Alternativ ist es ebenfalls möglich, den Abstand zwischen dem Ventilsitz 12 und den Vorsprüngen 30 in radialer Richtung zu ändern, wodurch der zwischen diesen eingeschlossene Hebel in seiner Größe geändert wird. Hierdurch ist ebenfalls eine Änderung des Öffnungsdrucks des Rückschlagventils 1 möglich.

Zur Montage sind die beiden Schlauchanschlussgehäuse 2 und 4 mittels eines innen liegenden Ringvorsprungs 34 am ersten Schlauchanschlussgehäuse 2 und mittels eines aussen liegenden Ringvorsprungs 36 am zweiten Schlauchanschlussgehäuse 4 ineinander greifend verbindbar. Nach Herstellung dieser Verbindung kann die endgültige Montage beispielsweise durch Ultraschallschweißen erfolgen.

Sämtliche aus der Beschreibung, den Ansprüchen und Zeichnungen hervorgehenden Merkmale und Vorteile der Erfindung, einschließlich konstruktiver Einzelheiten und räumlicher Anordnungen, können sowohl für sich als auch in beliebiger Kombination erfindungswesentlich sein.

### BEZUGSZEICHENLISTE

- 1 =: Rückschlagventil
- 2 =: erstes Schlauchanschlussgehäuse
- 4 =: zweites Schlauchanschlussgehäuse
- 6 =: Membranscheibe
- 8 =: Eingangsdurchlass
- 10 =: Einlassraum
- 12 =: Ventilsitz
- 14 =: Ringwulst
- 16 =: Ringnut
- 18 =: Ringnut
- 20 =: Öffnungen
- 21 =: Ringraum
- 22 =: Auslassraum
- 24 =: Ausgangsdurchlass
- 26 =: Wandung
- 28 =: Formation
- 30 =: Vorsprünge
- 32 =: Eingangsöffnung
- 34 =: Ringvorsprung
- 36 =: Ringvorsprung

## Patentansprüche

1. Rückschlagventil (1), insbesondere für medizinische Anwendungen, mit einem ersten Schlauchanschlussgehäuse (2) und einem zweiten Schlauchanschlussgehäuse (4) und einer zwischen den beiden Schlauchanschlussgehäusen (2, 4) angeordneten_{.} Membranscheibe (6) aus flexiblem Werkstoff, welche bei Überdruck in einem Eingangsdurchlass (8) im ersten Schlauchanschlussgehäuse (2) von einem ringförmigen, einen mit dem Eingangsdurchlass (8) verbundenen Eingangsraum (10) umgebenden Ventilsitz (12) abhebbar ist und die bei Überdruck in einem Ausgangsdurchlass (24) sicher und in Minimalzeiten auf den Ventilsitz (12) andrückbar ist, wobei die Membranscheibe (6) an ihrem äußeren Umfangsbereich mit einem Ringwulst (14) versehen ist, welcher in einander gegenüber liegenden Ringnuten (16, 18) der Schlauchanschlussgehäuse (2, 4) aufgenommen ist, wobei die Membranscheibe (6) radial außerhalb des Ventilsitzes (12) mit Öffnungen (20) versehen ist, die mit einem Auslassraum (22) verbunden sind, **dadurch gekennzeichnet, dass** auf der der Membranscheibe (6) gegenüber liegenden Wandung (26) des zweiten Schlauchanschlussgehäuses (4) eine an der dem Auslassraum (22) zugewandte Seite der Membranscheibe (6) anliegende Formation (28) vorgesehen ist, dass die Formation (28)
die Membranscheibe (6) in Richtung des Einlassraums (10) hält bzw. vorspannt, dass die Formation (28) innerhalb der durch den Ventilsitz (12) gegebenen Grenze, angeordnet ist, und dass der Öffnungsdruck des Ventils (1) durch den Abstand zwischen der Formation (28) und dem Ventilsitz (12) bestimmt ist.

2. Rückschlagventil nach Anspruch 1, **dadurch gekennzeichnet, dass** die Formation (28) mediendurchlässig ausgebildet ist.

3. Rückschlagventil nach Anspruch 1 oder 2, **dadurch gekennzeichnet, dass** die Formation (28) durch eine Anzahl von Vorsprüngen (30) gebildet ist, welche kronenförmig die Eingangsöffnung (32) des Auslassdurchlasses (24) vom Auslassraum (22) umgeben.

4. Rückschlagventil nach Anspruch 3, **dadurch gekennzeichnet, dass** die Vorsprünge (30) einstückig mit dem zweiten Schlauchanschlussgehäuse (4) ausgebildet sind.

## Claims

1. Non-return valve (1), particularly for medical applications, with a first hose connection housing (2) and a second hose connection housing (4) and, arranged between the two hose connection housings (2, 4), a diaphragm disc (6) which is made of flexible material and which, with an overpressure in an inlet channel (8) in the first hose connection housing (2), can be lifted from an annular valve seat (12) that surrounds an inlet space (10) connected to the inlet channel (8), and which, with an overpressure in an outlet channel (24), can be pressed onto the valve seat (12) safely and in minimal times, which diaphragm disc (6) is provided at its outer circumferential area with an annular bead (14) which is received in mutually opposite annular grooves (16, 18) of the hose connection housings (2, 4), and which diaphragm disc (6) is provided, radially outside the valve seat (12), with openings (20) connected to an outlet space (22), **characterized in that**, on the wall (26) of the second hose connection housing (4) lying opposite the diaphragm disc (6), a formation (28) is provided which bears on that side of the diaphragm disc (6) directed towards the outlet space (22), **in that** the formation (28) holds or pretensions the diaphragm disc (6) in the direction of the inlet space (10), **in that** the formation (28) is arranged within the limit defined by the valve seat (12), and **in that** the opening pressure of the valve (1) is determined by the distance between the formation (28) and the valve seat (12).

2. Non-return valve according to Claim 1, **characterized in that** the formation (28) is designed to be permeable to media.

3. Non-return valve according to Claim 1 or 2, **characterized in that** the formation (28) is formed by a number of projections (30) which, in the shape of a crown, surround the inlet opening (32) of the outlet channel (24) from the outlet space (22).

4. Non-return valve according to Claim 3, **characterized in that** the projections (30) are formed in one piece with the second hose connection housing (4).

## Revendications

1. Clapet anti-retour (1), notamment à usage médical, comprenant un premier boîtier de raccord de tube (2) et un deuxième boîtier de raccord de tube (4) et un disque de membrane (6) en matériau flexible disposé entre les deux boîtiers de raccord de tube (2, 4), lequel, en cas de surpression dans un passage d'entrée (8) dans le premier boîtier de raccord de tube (2), peut être soulevé d'un siège de clapet (12) de forme annulaire, entourant un espace d'entrée (10) connecté au passage d'entrée (8), et qui, en cas de surpression dans un passage de sortie (24) peut être pressé de manière sûre et dans un temps minimum sur le siège de clapet (12), le disque de membrane (6) étant pourvu sur sa région périphérique extérieure d'un bourrelet annulaire (14), qui est reçu dans des rainures annulaires opposées (16, 18) des boîtiers de raccord de tube (2, 4), le disque de membrane (6) étant pourvu radialement à l'extérieur du siège de clapet (12) d'ouvertures (20) qui sont connectées à un espace de sortie (22), **caractérisé en ce qu'**une formation (28) s'appliquant contre le côté du disque de membrane (6) tourné vers l'espace de sortie (22) est prévue sur la paroi (26) du deuxième boîtier de raccord de tube (4) opposée au disque de membrane (6), **en ce que** la formation (28) retient ou précontraint le disque de membrane (6) dans la direction de l'espace d'entrée (10), **en ce que** la formation (28) est disposée à l'intérieur de la limite donnée par le siège de clapet (12), et **en ce que** la pression d'ouverture du clapet (1) est déterminée par la distance entre la formation (28) et le siège de clapet (12).

2. Clapet anti-retour selon la revendication 1, **caractérisé en ce que** la formation (28) est réalisée de manière à laisser passer des milieux.

3. Clapet anti-retour selon la revendication 1 ou 2, **caractérisé en ce que** la formation (28) est formée par un certain nombre de saillies (30) qui entourent en forme de couronne l'ouverture d'entrée (32) du passage de sortie (24) de l'espace de sortie (22).

4. Clapet anti-retour selon la revendication 3, **caractérisé en ce que** les saillies (30) sont réalisées d'une seule pièce avec le deuxième boîtier de raccord de tube (4).
